# EUROPÄISCHE PATENTSCHRIFT

(11) **EP 0 549 593 B1**
(45) Veröffentlichungstag und Bekanntmachung des Hinweises auf die Patenterteilung: **09.10.1996**
(21) Anmeldenummer: 91913842.0
(22) Anmeldetag: 31.07.1991
(51) Int. Cl.: C07C 62/24, C07C 51/487, C07C 51/493, C07C 51/353, C07B 55/00

(54) **VERFAHREN ZUR RACEMISIERUNG VON NICHTRACEMISCHEN 3-OXOCYCLOPENTAN- BZW. -HEXANCARBONSÄUREN ODER IHREN ESTERN MIT C 1-C 6-ALKOHOLEN**
METHOD FOR RACEMIZATION OF NON-RACEMIC 3-OXOCYCLOPENTANE OR 3-OXOCYCLOHEXANE CARBOXYLIC ACIDS AND THEIR ESTERS WITH C 1-C 6 ALCOHOLS
PROCEDE DE RACEMISATION D'ACIDES 3-OXOCYCLOPENTANCARBOXYLIQUES OU HEXANCARBOXYLIQUES NON RACEMIQUES OU DE LEURS ESTERS AVEC DES ALCOOLS C 1-C 6

(30) Priorität: 04.08.1990 DE 4024836
(43) Veröffentlichungstag der Anmeldung: 07.07.1993
(73) Patentinhaber: BOEHRINGER INGELHEIM INTERNATIONAL GmbH, D-55216 Ingelheim am Rhein (DE); BOEHRINGER INGELHEIM KG, D-55216 Ingelheim (DE)
(72) Erfinder: SOBOTTA, Rainer, D-6507 Ingelheim (DE)
(86) Internationale Anmeldenummer: EP9101434
(87) Internationale Veröffentlichungsnummer: WO9202483

(56) Entgegenhaltungen:
- Patent Abstracts of Japan, Band 13, Nr. 169, 21. April 1989 (C-587)[3517], & JP, A, 6446, (SUMITOMO CHEM. CO. LTD) 5. Januar 1989

## Beschreibung

Die Erfindung betrifft ein neues Verfahren, mit dem nichtracemische 3-Oxocyclopentan- bzw. 3-Oxocyclohexancarbonsäure oder ihre Ester mit niederen Alkoholen zu den entsprechenden racemischen Säuren bzw. Estern racemisiert werden können.

Beispielsweise für die Herstellung von Hetrazepinen, die als Arzneistoffe verwendet werden können, werden reine Enantiomere von 3-Oxocycloalkancarbonsäuren benötigt. Da bei der Synthese dieser Säuren Racemate entstehen, ist eine Racematspaltung erforderlich. Damit auch das ungeeignete Enantiomere verwertet werden kann, muß es racemisiert und erneut der Racematspaltung zugeführt werden.

Die Erfindung löst nun die Aufgabe, Enantiomere bzw. nichtracemische Enantiomerengemische von 3-Oxocycloalkancarbonsäuren oder ihre Ester mit niederen Alkoholen in das Racemat umzuwandeln.

Auf diese Weise können insbesondere die bei der oben erwähnten Racematspaltung übrigbleibenden unverwertbaren Enantiomeren bzw. nichtracemischen Enantiomerengemische zu einem nützlichen Produkt verarbeitet werden.

Mit üblichen Methoden, z.B. durch Einwirkung von Basen auf nicntracemische 3-Oxocycloalkancarbonsäuren oder ihre Ester ist die Racemisierung nicht möglich; es entstehen überwiegend Kondensationsprodukte.

Es wurde nun ein Verfahren gefunden, mit dem sich nichtracemische 3-Oxocyclopentan- bzw. 3-Oxocyclohexancarbon säuren oder ihre Ester mit C₁-C₆-Alkoholen überraschender weise in das entsprechende Racemat überführen lassen.

Das Verfahren ist dadurch gekennzeichnet, daß man die Säu ren oder Ester mit einem Orthoameisensäure-C₁-C₆-alkyl ester in Gegenwart katalytischer Mengen einer starken Säure in einem C₁-C₆-Alkohol als Reaktionsmedium ketalisiert und (falls nicht schon ein Ester vorliegt) zugleich verestert, das erhaltene Produkt durch Einwirkung eines Alkoholats racemisiert, anschließend durch Behandlung mit verdünnter Säure zur racemischen Säure bzw. zum racemischen Ester hydrolysiert und die racemischen Verbindungen in üblicher Weise isoliert.

Die Teilschritte werden zweckmäßig ohne Isolierung von Zwischenprodukten durchgeführt.

Als Alkohol benutzt man einen aliphatischen Alkohol mit 1-6 C-Atomen, vorzugsweise Methanol, Ethanol, Propanol. Zweck mäßig ist die Verwendung von Orthoameisensäuren und Alkoholaten (z.B. Natrium- oder Kaliumalkoholaten), die sich von demselben Alkohol ableitet, der als Reaktionsmedium dient. Als Katalysatoren werden die für diese Zwecke gebräuchlichen Säuren (starke Mineralsäuren wie Schwefelsäure, Salzsäure; Toluolsulfonsäure, Methansulfonsäure) angewendet.

Die Herstellung des Ketals erfolgt vorzugsweise bei der Siedetemperatur des Reaktionsgemischs. Die Hydrolyse der Ketalfunktion (zum Ester) wird bei Raumtemperatur oder unter Kühlung vorgenommen. Soll auch die Estergruppe hydrolysiert werden, so geschieht dies zweckmäßig bei Rückflußtemperatur.

Je ein Beispiel für die Durchführung der Verfahrensvarianten, die der Fachmann gewünschtenfalls ohne Schwierigkeit modifizieren kann, ist nachstehend beschrieben.

### Beispiel 1

### Racemischer 3-Oxocyclopentancarbonsäuremethylester

Zu einer Lösung von 128,2 g (1 mol) nichtracemischer 3-Oxocyclopentancarbonsäure und 11,4 g (0,06 mol) p-Toluolsulfonsäure-Hydrat in 380 ml Methanol werden unter Rühren 318,36 g (3 mol) Trimethylorthoformiat innerhalb von 5 - 10 Minuten zudosiert. Anschließend wird erhitzt und im Verlauf von 25 Minuten ein Gemisch aus Ameisensäuremethylester und Methanol abdestilliert (ca. 185 g Destillat). Danach werden 29 g 30 %ige Natriummethylatlösung (0,16 mol) zugegeben und das Reaktionsgemisch 2 Stunden am Rückfluß zum Sieden erhitzt; Methanol wird abdestilliert, der Rückstand auf 10°C abgekühlt und durch Zugabe von 230 ml Methylenchlorid und 200 ml Wasser wieder in Lösung gebracht. Durch Zugabe von 5%iger Schwefelsäure wird das Gemisch auf pH 1,4 eingestellt und 30 Minuten bei 10°C gerührt. Die Methylenchloridphase wird abgetrennt und die wäßrige Phase nochmals mit 100 ml Methylenchlorid extrahiert. Die vereinigten organischen Phasen werden mit 50 ml Wasser gerührt, abgetrennt und das Lösungsmittel im Vakuum abdestilliert. Der Rückstand wird im Vakuum fraktioniert.

Hauptfraktion: 131,4 g; Kp_{23mbar}: 110°C; 92 % d.Th. Eine Probe im Polarimeter zeigt einen Drehwinkel von 0° an.

### Beispiel 2

### Racemische 3-Oxocyclohexancarbonsäure

Wie im Beispiel 1 beschrieben, werden 7,11 g (0,05 mol) nichtracemische 3-Oxocyclohexancarbonsäure mit 18,9 g (0,178 mol) Trimethylorthoformiat in Gegenwart von 0,57 g (0,03 mol) 30%iger Natriummethylatlösung racemisiert.
Die Hydrolyse zur racemischen Ketosäure erfolgt nach dem Abdestillieren des Lösungsmittels. Zum Rückstand werden 85 g 2N Salzsäure gegeben und das Gemisch 6 Stunden am Rückfluß zum Sieden erhitzt. Anschließend wird im Vakuum auf einen Rückstand von 21 g eingeengt und dieser nach Abkühlung einmal mit 50 ml und zweimal mit je 20 ml Chloroform extrahiert. Die vereinigten organischen Phasen werden im Vakuum vom Lösungsmittel befreit. Eine Probe des Rückstands zeigt im Polarimeter keinen Drehwinkel an.
Ausbeute: 6,7 g (94,2 % d.Th.)

## Patentansprüche

1. Verfahren zur Racemisierung von nichtracemischen 3-Oxocyclopentan- bzw. -hexancarbonsäuren oder ihren Estern mit C₁-C₆-Alkoholen, dadurch gekennzeichnet, daß man diese Säuren oder Ester mit einem Orthoameisensäure-C₁-C₆-alkylester in Gegenwart Katalytischer Mengen einer starken Säure in einem C₁-C₆-Alkohol als Reaktionsmedium ketalisiert und (falls nicht schon ein Ester vorliegt) zugleich verestert, das erhaltene Produkt durch Einwirkung in eines Alkoholats racemisiert, anschließend durch Behandlung mit verdünnter Säure zur racemischen Säure bzw. zum racemischen Ester hydrolysiert und die racemischen Verbindungen in üblicher Weise isoliert.

2. Verfahren nach Anspruch 1, dadurch gekennzeichnet, daß man zur Racemisierung ein Natrium- oder Kaliumalkoholat verwendet.

3. Verfahren nach Anspruch 1 oder 2, dadurch gekennzeichnet, daß als Reaktionsmedium ein C₁-C₃-Alkohol verwendet wird und der Orthoester sowie das Alkoholat sich von demselben Alkohol ableiten.

4. Verfahren nach Anspruch 3, dadurch gekennzeichnet, daß der Alkohol Methanol oder Ethanol ist.

5. Verfahren nach einem der vorhergehenden Ansprüche, dadurch gekennzeichnet, daß die Hydrolyse zur Gewinnung der racemischen Säuren in der Siedehitze, die Hydrolyse zur Gewinnung der racemischen Ester bei Raumtemperatur oder unter leichter Kühlung erfolgt.

## Claims

1. A process for the racemization of non-racemic 3-oxocyclopentane- or -hexanecarboxylic acids or their esters with C₁₋₆-alcohols, characterized in that those acids or esters are ketalised with a C₁₋₆-alkyl orthoformate in the presence of a catalytic amount of a strong acid, in a C₁₋₆-alcohol as reaction medium, and (if there is no ester already present) esterified at the same time, the resulting product is racemized by the action of an alkoxide, then hydrolyzed by treatment with dilute acid to give the racemic acid or racemic ester and the racemic compounds are isolated according to conventional methods.

2. Process according to claim 1, characterized in that a sodium or potassium alkoxide is used for the racemization.

3. Process according to claim 1 or 2, characterized in that a C₁₋₃-alcohol is used as the reaction medium and that the orthoester as well as the alkoxide are derived from the same alcohol.

4. Process according to claim 3, characterized in that the alcohol is methanol or ethanol.

5. Process according to one of the preceding claims, characterized in that the hydrolysis for the production of the racemic acids is carried out at boiling temperature, the hydrolysis for the production of the racemic esters is carried out at ambient temperature or with slight cooling.

## Revendications

1. Procédé pour racémiser des acides 3-oxocyclopentane- ou -hexanecarboxyliques non racémiques ou leurs esters avec des alcools en C₁-C₆, caractérisé en ce que l'on cétalise ces acides ou esters avec un orthoformiate d'alkyle en C₁-C₆ en présence de quantités catalytiques d'un acide fort dans un alcool en C₁-C₆ comme milieu réactionnel et (si un ester n'est pas déjà présent) on les estérifie en même temps, on racémise le produit obtenu par action d'un alcoolate, puis on l'hydrolyse en l'acide racémique ou en l'ester racémique par traitement avec un acide dilué et on isole les composés racémiques de manière habituelle.

2. Procédé selon la revendication 1, caractérisé en ce que l'on utilise pour la racémisation un alcoolate de sodium ou de potassium.

3. Procédé selon la revendication 1 ou 2, caractérisé en ce qu'un alcool en C₁-C₃ est utilisé comme milieu réactionnel et l'orthoester et l'alcoolate dérivent du même alcool.

4. Procédé selon la revendication 3, caractérisé en ce que l'alcool est le méthanol ou l'éthanol.

5. Procédé selon l'une des revendications précédentes, caractérisé en ce que l'hydrolyse pour l'obtention des acides racémiques a lieu à la température d'ébullition, l'hydrolyse pour l'obtention des esters racémiques a lieu à la température ambiante ou sous léger refroidissement.
